# EUROPEAN PATENT SPECIFICATION

(11) **EP 1 001 759 B1**
(45) Date of publication and mention of the grant of the patent: **20.07.2005**
(21) Application number: 97941503.1
(22) Date of filing: 10.09.1997
(51) Int. Cl.: A61K 31/20, A61K 31/045, A61K 9/107, A61K 47/10, A61P 31/12

(54) **VIRAL INHIBITION BY LONG-CHAIN ALCOHOLS, ALKANES, FATTY ACIDS AND AMIDES**
LANGKETTIGE ALKOHOLE, ALKANE, FETTSÄUREN UND AMIDE ALS VIRENINHIBITOREN
INHIBITION VIRALE AU MOYEN D'ALCOOLS, ALCANES, ACIDES GRAS ET AMIDES A CHAINE LONGUE

(30) Priority: 17.09.1996 US 715009; 22.08.1997 US 916624
(43) Date of publication of application: 24.05.2000
(60) Divisional application: 05075320.1
(73) Proprietor: AVANIR PHARMACEUTICALS, San Diego, California 92121 (US)
(72) Inventor: KATZ, David, H., La Jolla, CA 92037 (US); POPE, Laura, E., Carlsbad, CA 92009 (US); KHALIL, Mohammed, H., San Diego, CA 92130 (US); MARCELLETTI, John, F., San Diego, CA 92120 (US); KATZ, Lee, R., La Jolla, CA 92037 (US)
(74) Representative: W.P. Thompson & Co.
(86) International application number: PCT/US1997/016007
(87) International publication number: WO 1998/011887

(56) References cited:
- EP-A- 0 526 666
- US-A- 4 880 634

## Description

### Field of the Invention

This invention relates to compositions for use in therapeutic treatment of viral infections and skin inflammation. The compositions comprise unsaturated long chain alcohols, particularly a nonionic surfactant and stearyl alcohol erucyl alcohol, erucamide, brassidyl alcohol, arachidyl alcohol, *n*-docosanol, *n*-docosane, *n*-docosanoic acid, or stearic acid or mixtures thereof as active ingredients.

### Background of the Invention

Viral infections cause considerable discomfort, disease and can be fatal. Viruses such as herpes simplex viruses (HSV-1 and HSV-2), cytomegalovirus (CMV), Epstein-Barr virus (EBV), varicella zoster virus (VZV), influenza viruses, human lymphotrophic viruses (e.g., HTLV-1) and human immunodeficiency viruses (e.g., HIV-1) result in significant morbidity and mortality. HSV-1 and HSV-2 are associated with inflammation and lesions of the skin and mucosal membranes, including cold sores, fever blisters and genital herpes lesions. VZV causes shingles and EBV is associated with mononucleosis. Influenza viruses cause flu symptoms and can be fatal. HIV causes acquired immunodeficiency which debilitates and kills infected individuals. Although these viruses may remain latent in some cells and for varying periods of time, generally viral replication results in irreversible destruction of the infected cell producing different clinical manifestations of the diseases they cause.

Antiviral and anti-inflammatory activities of aliphatic alcohols having from 20 to 32 carbons are known in the art as disclosed in U.S. Patent No. 4,874,794, U.S. Patent No. 5,071,879, U.S. Patent No. 5,168,219, U.S. Patent No. 5,194,451 and U.S. Patent No. 5,534,554. Compositions containing aliphatic alcohols and related compounds having therapeutic activities are disclosed therein.

A C22 aliphatic alcohol, *n*-docosanol, suspended in a surfactant exhibits potent antiviral activity against viruses including herpes simplex virus, HIV-1 and respiratory syncytial virus *in vitro* and Friend virus *in vivo* (Katz, D. H., et al., *Proc. Natl. Acad. Sci. USA* 88:10825-10829, 1991; U.S. Patent No. 5,534,554). Although the mechanism for this viral inhibition is unknown, *n*-docosanol does not inactivate the virus directly and thus is unlike C10 to C18 unsaturated alcohols that exhibit detergent-like antiviral activity (Katz, D. H., et al., *Proc. Natl. Acad. Sci. USA* 88:10825-10829, 1991; Snipes, W. et al., *Antimicrob. Agents Chemother*. 11:98-104, 1977). Progressive binding and uptake of *n*-docosanol by cells may account for its antiviral activity because pre-incubation of cells with the alcohol produces optimal antiviral activity. During incubation, 70% of the cell-associated *n*-docosanol is found in cell membranous components and the remainder is associated with soluble cell fractions (Katz, D. H., et al., *Proc. Natl. Acad. Sci. USA* 88:10825-10829, 1991). Cell membrane incorporation of *n*-docosanol does not inhibit virus binding to the cell surface. Instead, early viral protein synthesis is inhibited more than 80% and viruses do not localize to nuclei (Marcelletti, J. F. et al., *Drugs of the Future* 17(19): 879-882, 1992). Although intracellular metabolic conversions of *n*-docosanol may account for its antiviral activity (Katz, D. H. et al., *Annals N.Y. Acad. Sciences,* 724:472-488, 1994), the alcohol is not cytotoxic in concentrations up to 300 mM.

Inactivation of viruses has been reported using C 14 to C20 unsaturated long chain alcohols having one to four unsaturated bonds. The most effective was *y*-linolenyl alcohol, a C18 alcohol with double bonds at positions 6, 9 and 12; whereas a C18 alcohol with one *cis* double bond and a C20 alcohol with four double bonds were significantly lass effective (Sands et aL, Antimicrob. Agents & Chemother. 15:67-73, 1979). Compositions containing oleic acid (C18, one double bond) have been reported as effective for anti-herpes virus agents (PCT patent application WO 9602244A1).

Some compounds that are structurally related to long-chain aliphatic alcohols also have been associated with antiviral activity. For example, U.S. Patent No. 4,513,008 discloses the virucidal activity of C20 to C24 linear polyunsaturated acids, aldehydes or alcohols having five to seven double bonds. Compounds having a long chain fatty acyl group, containing at least three or four unsaturated bonds, attached to a nucleoside or nucleoside analogue are disclosed as antiviral treatments in U.S. Patent No. 5,216,142. Related U.S. Patent No. 5,276,020 discloses antiviral compounds having a C16, C18 or C20 long chain fatty acid group attached to a nucleoside analogue and a method of treating virus infection using these compounds.

Biological activity useful for treating tumors, protozoal and fungal diseases, autoimmune disease and bone marrow damage has been attributed to phospholipids having erucyl- and brassidyl-side chains, such as erucylphosphocholine (U.S. Patent No. 5,436,234).

Although same long chain fatty alcohols and fatty acids affect cellular growth, such effects are presently ill-defined. For example, *n*-hexacosanol, a C26 alcohol, promotes neuronal growth whereas other long chain fatty *n*-alcohols containing 16, 20, 22, 24 and 30 carbon atoms do not (Borg, J. et al., *FEBS Lett*. 213(2):406-410, 1987). Docosahexaenoic acid, a C22 fatty acid having six double bonds, is concentrated in the central nervous system and the retina although its physiological role has not been defined (Bazan, N., *Prog. Clin. Biol. Res.* 312:95-112, 1989).

Antiviral activity has been reported for liposomal AL721, a mixture of neutral glycerides, phophatidylcholine and phosphatidylethanolamine (Antonian, L et aL, *Neurosci. Biobehav. Rev*. 11:399-413, 1987). Antimicrobial compositions for topical treatment containing a C15 glycerol monoester of lauric acid or a polyhydric alcohol monoester of lauric acid with a mixture of fatty acids (C10 capric and C8 caprylic acids) are disclosed in U.S. Patent No. 5,208,257.

A method of preventing or reducing skin irritation by applying a protective agent containing polymers of C12 to C26 fatty acids prior to exposure to an allergenic agent is disclosed in U.S. Patent No. 4,076,799. The preferred polymers have two to four carboxy or carboxyl salt groups, preferably the triethanolamine salt of dimerized linoleic acid or its saturated derivative. Other anti-inflammatory polymers containing aromatic heterocyclic residues or acyl residues in homopolymers or heteropolymers (e.g., vinyl esters of C8 to C18 fatty acids; m.w. 2,000 to 1,000,000) and having greater activity than the component monomers have been disclosed in U.S. Patent No. 3,946,035.

Therapeutic treatment of herpes lesions using topically administered compositions containing an anesthetic, a surfactant and a topical carrier is disclosed in U.S. Patent No. 5,380,754. A method of treating inflammation by topically applying ethyl-*cis*,*cis*(9,12)octadecadienoate (ethyl linoleate) is disclosed in U.S. Patent No. 4,025,645 as a cold sore treatment.

The present invention discloses antiviral and cytotoxic effects of compounds related to long-chain aliphatic alcohols, including alkanes, alcohols, amides and long-chain fatty acids, and particularly compounds related to *n*-docosanol. These related compounds having antiviral activity include *n*-docosane, *n*-docosanoic acid, stearic acid, erucyl alcohol, erucamide and brassidyl alcohol. Moreover, the optimal ratio of surfactant to active ingredient for formulating an effective antiviral and/or cytotoxic suspension with these compounds or with *n*-docosanol is disclosed. These compounds and formulations are useful in antiviral preventive compositions and treatment therapeutics.

### Summary of the Invention

According to the invention, there is provided a composition comprising a mixture of about 1:1 (w:w) to about 10:1 (w:w) of a nonionic surfactant and an active ingredient in a pharmaceutically acceptable diluent or carrier, wherein the nonionic surfactant is an octoxynol and wherein the active ingredient is stearyl alcohol, erucyl alcoholic, brassidyl alcohol, n-docosanol, arachidyl alcohol, n-docosane, n-docosanoic acid erucamide, stearic acid, or mixtures thereof. In one embodiment, the mixture comprises about 5:1 (w:w) to about 10:1 (w:w) of the nonionic surfactant and the active ingredient. In another embodiment, the mixture comprises about 4:1 (w:w) to about 10:1 (w:w) of the nonionic surfactant and *n*-docosanol. In one embodiment, the mixture comprises about 4:1 (w:w) to about 10:1 (w:w) of the nonionic surfactant and stearic acid.

In one embodiment, the surfactant comprises a mixture of non-ionic surfactants including e.g. a block polymer comprising a polyoxyalkylene derivative of propylene glycol having a molecular weight of about 25,000, or a block polymer comprising ethylene oxide and propylene oxide having a molecular weight of about 8,400, or *n*-docosanol. In one embodiment, the nonionic surfactant is octoxynol-9, octoxynol-10 or a combination thereof. In another embodiment, the mixture comprises about 5% to about 20% (w:w) stearic acid and further comprises a sugar based stearate. Another preferred embodiment is the mixture that comprises about 10% to about 12% (w:w) *n*-docosanol and further comprises a sugar based stearate. In one preferred embodiment, the mixture consists essentially of a suspension of a nonionic surfactant and steric acid in a pharmaceutically acceptable diluent or carrier. One embodiment of the invention provides for use of a composition comprising a mixture according to any of the above embodiments for inhibiting cell growth or proliferation. Another embodiment of the invention provides for use of a composition comprising any of the above described mixtures for prevention or treatment of a viral infection, preferably for prevention or treatment of a viral infection caused by a herpes simplex virus, cytomegalovirus, Epstein-Barr virus, varicella zoster virus, influenza virus, human lymphotrophic virus, human immunodeficiency virus, papilloma virus or respiratory syncytial virus. One embodiment of the invention provides for use of any one of the above-described mixtures for relieving skin or membrane inflammation. Another embodiment of the invention is use of a composition comprising any of the above-described mixtures for preparation of a medicament suitable for topical, transmembrane or parenteral application.

### Brief Description of the Drawings

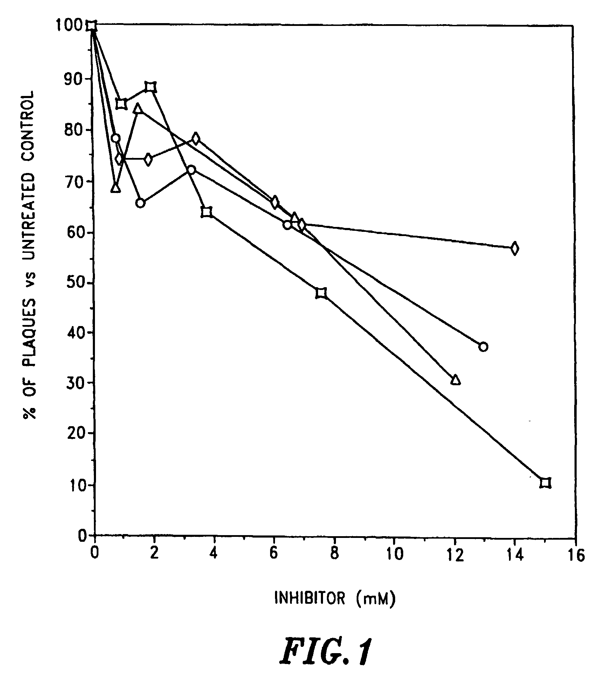

FIG. 1 is a diagram showing inhibition of HSV-2 plaque formation in Vero cells *in vitro* by suspensions of *n*-docosanol (C22, ■), *n*-tetracosanol (lignoceryl) alcohol (C24, ◇), *n*-hexacosanol (C26, ●) and *n*-octacasanol (C28,Δ) at the concentrations shown on the X-axis (data is percentage of plaques observed compared to control cultures exposed to surfactant suspensions lacking long-chain alcohol).

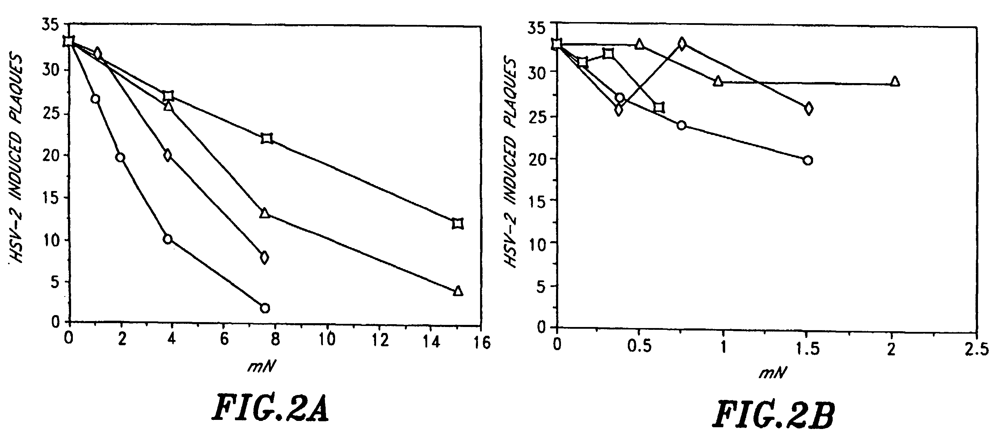

FIG. 2A is a diagram showing that increasing the ratio of surfactant to *n*-docosanol decreases viral plaque production when Vero cells are incubated with the suspension for 12 hours before adding HSV-2 virus; the surfactant:*n*-docosanol ratios were 1:1 (■), 3:1 (Δ), 5:1 (◆) and 10:1 (○).
FIG. 2B shows the corresponding controls as in FIG. 2A using the same concentration of surfactant in suspension as for each surfactant:alcohol ratio shown in FIG. 2A but without *n*-docosanol (using the same symbols as in FIG. 2A).

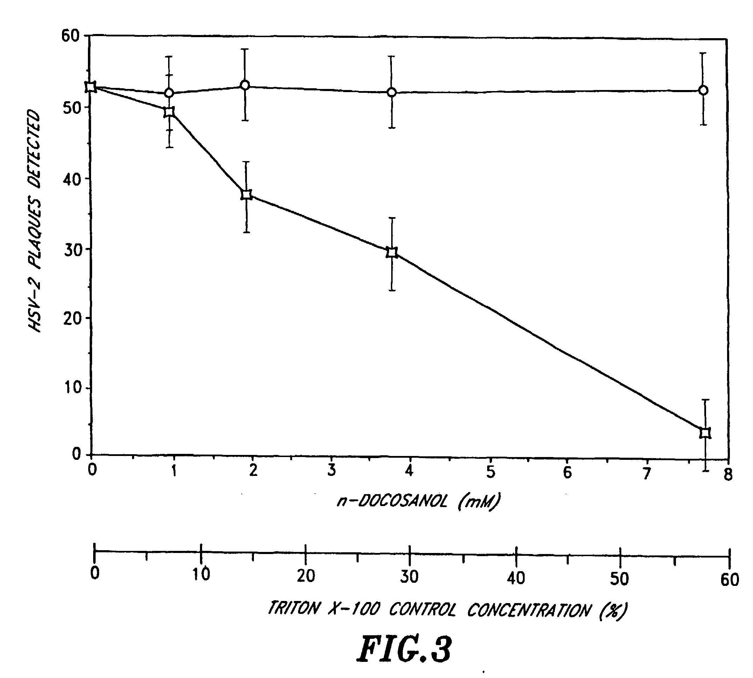

FIG. 3 is a diagram showing that octoxynol surfactant suspensions of *n*-docosanol (■) inhibit HSV-2 plaque formation in Vero cells incubated with the suspension and HSV-2 for 48 hours with increasing inhibition correlated with increasing concentration of *n*-docosanol, whereas control cultures incubated with HSV-2 and octoxynol surfactant (○) showed no inhibition (i.e., equivalent to untreated controls having about 50 plaques/well); bars above and below the data points show the standard deviation for duplicate samples.

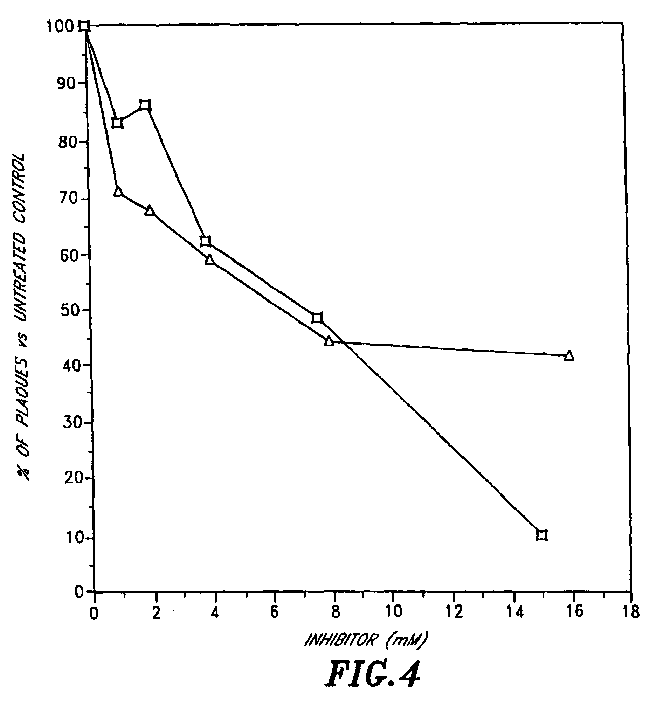

FIG. 4 is a diagram showing that suspensions of surfactant/*n*-docosanol (■) and surfactant/*n*-docosane (Δ) inhibit HSV-2 viral plaque formation in cultured Vero cells incubated with the compounds for 12 hours before the addition of HSV-2.

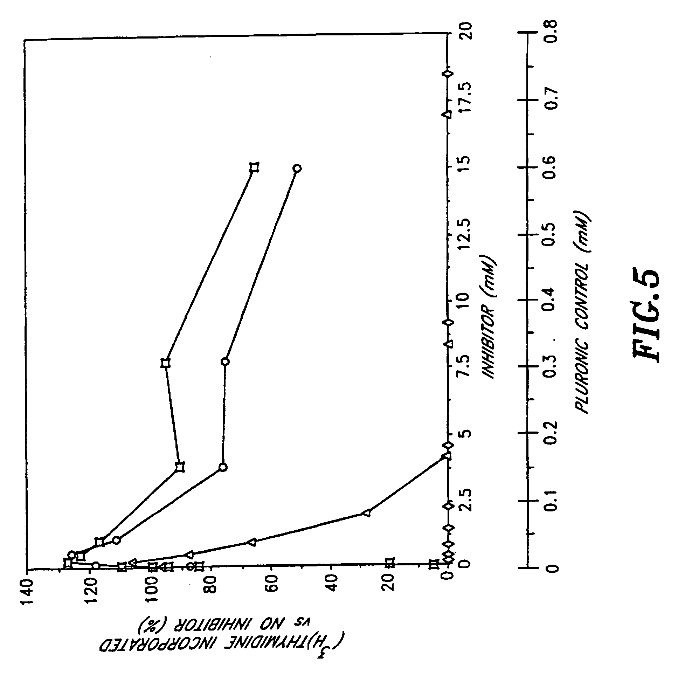

FIG. 5 is a diagram showing that suspensions of stearyl alcohol (C18, ◆) and arachidyl alcohol (C20, Δ) are toxic to cuhured B-cell tumor calls incubated for 48 hours with the suspensions at the concentrations indicated on the X-axes compared to controls incubated with suspensions of surfactant without alcohol (○) or surfactant/*n*-docasanol (C22, ■) as determined by ³H-thymidine incorporation into DNA (data is the percentage of controls incubated with media only).

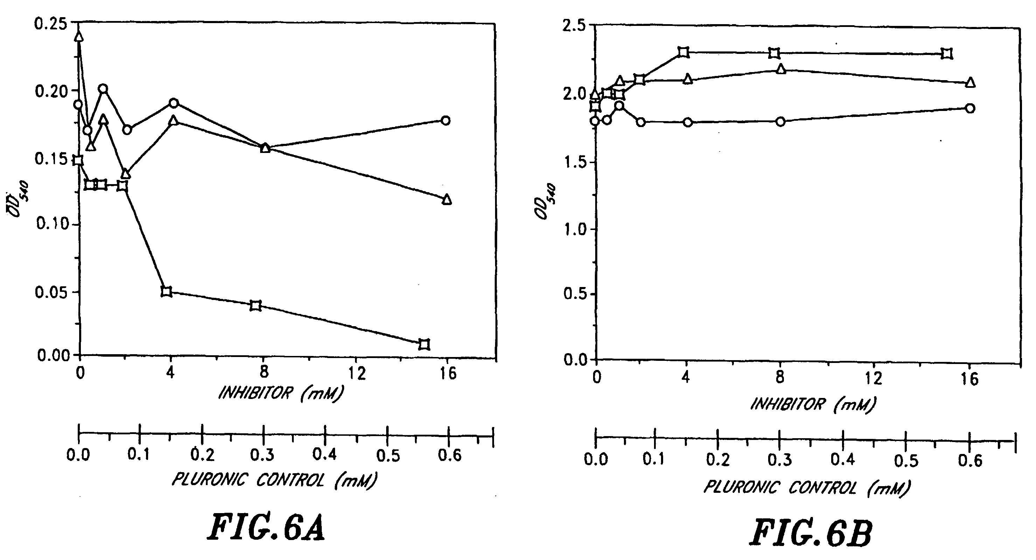

FIG. 6A and FIG. 6B diagrammatically show the cellular antiproliferative effects of suspensions of surfactant/*n*-docosanol (■) on foreskin fibroblasts compared to cells incubated with suspensions of surfactant/*n*-docosane (Δ) or with controls incubated with a surfactant suspension without active ingredient (○) at the concentrations shown on the X-axes (averages of duplicate assays quantitated after 96 hours incubation of cells inoculated at 1,000 cells/well (FIG. 6A) or 30,000 cells/well (FIG. 6B) in 96-well plates).

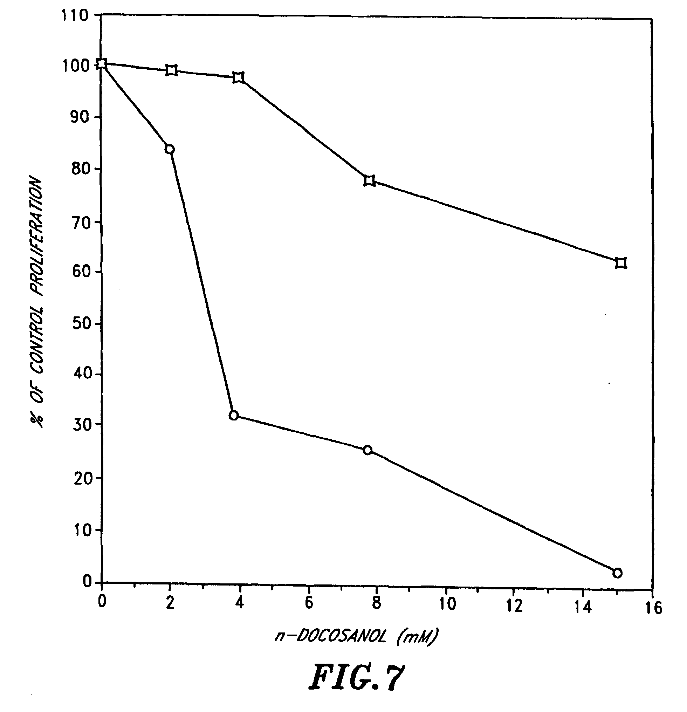

FIG. 7 is a diagram showing the time dependence of cellular antiproliferative effects of surfactant/*n*-docosanol suspension after 72 hr (■) and 96 hr (○) incubation using the methods as described for FIG. 6A.

### Detailed Description of the Preferred Embodiment

Methods of synthesis of *n*-docosanol and erucyl alcohol (*cis*-13-docosen-1-ol) are known to those skilled in the art (e.g., see U.S. Patent No. 4,186,211). Stearyl alcohol can be synthesized according to the method of Brown et al. (*J. Am. Chem. Soc.* 78:2582, 1956). Methods of synthesis of alkanes, aliphatic alcohols, amides and aliphatic acids are well known in the art (e.g., see A. Streitwieser, Jr. & C.H. Heathcock, Introduction to Organic Chemistry, 2nd ed., Macmillan Publishing Co., New York, NY, 1981, at pages 160, 243-247, 303-307, 311-312, 315-317, 401-406, 447-053, 515-516, 544, 548-555, 604-605, 670, 753-754 and 950).

Compositions of this invention suitable for use in preventing or treating viral infections comprise an active ingredient or combination of compounds as the active ingredient, selected from a group consisting of saturated aliphatic alcohols, mono-unsaturated aliphatic alcohols, aliphatic alkanes, mono-unsaturated aliphatic amides and aliphatic acids having a carbon chain length of 18 to 28 carbons (C18 to C28). The preferred composition includes as an active ingredient stearyl alcohol, erucyl alcohol, erucamide, brassidyl alcohol, arachidyl alcohol, *n*-docosane, *n*-docosanoic acid and stearic acid, or mixtures thereof, preferably erucyl alcohol, erucamide, brassidyl alcohol, arachidyl alcohol, *n*-docosane, *n*-docosanoic acid, stearic acid, or mixtures thereof combined with a surfactant.
The active ingredients comprise about 0.1% to about 50% by weight of the final composition, preferably 1% to 10% by weight. The optimum antiviral activity of the active ingredients depends on the ratio of surfactant to active ingredient which may range from 1:1 (w:w) to 10:1 (w:w), and preferably is 5:1 (w:w).

Methods of suspending aliphatic alcohols, long-chain fatty acids and long-chain alkanes are well known in the art. One suitable method of making such a suspension is dilution of a nonionic detergent surfactant to 1 to 100 mglml in water or an aqueous solution such as a physiological saline solution and heating the solution (e.g., 37°C to 50°C). The active ingredient is then added to this surfactant solution to produce the desired final concentration of active ingredient and the combination is mixed (e.g., rotary or reciprocal mixing, stirring or sonicating), to produce a suspension of globular particles (about 0.1 µ to 100 µ average size). Other acceptable carriers include emulsions (oil-in-water or water-in-oil), solutions, creams, lotions, ointments, foams, gels and aerosols, all of which can be prepared using well-known methods.

The active agents and surfactants are combined with a carrier that is physiologically compatible with the skin and membrane tissue of a human or animal to which it is administered. That is, the carrier is substantially inactive except for surfactant properties used in making a suspension of the active ingredients. The compositions may include other physiologically active constituents that do not interfere with the efficacy of the saturated aliphatic alcohols, mono-unsaturated aliphatic alcohols, aliphatic alkanes and aliphatic acids. An exemplary composition is disclosed in U.S. Patent No. 3,592,930.

Suitable carriers include aqueous and oleaginous carries such as, for example, white petrolatum, isopropyl myristate, lanolin or lanolin alcohols, mineral oil, sorbitan mono-oleate, propylene glycol, cetylstearyl alcohol (together or in various combinations), with a detergent (e.g., polyoxyl stearate or sodium lauryl sulfate) and mixed with water to form a lotion, gel, cream or semi-solid composition. Other suitable carriers comprise mixtures of emulsifiers and emallients with solvents such as sucrose stearate, sucrose cocoate, sucrose distearate, mineral oil, propylene glycol, 2-ethyl-1,3-hexanediol, polyoxypropylene-15-stearyl ether and water. Preservatives may also be included in the carrier including mothylparabon, propylparaben, benzyl alcohol and ethylene diamine tetraacetate salts. Dilute suspensions without thickeners are most suitable for delivery to skin surfaces as aerosol sprays, using well known methods of delivery. The composition may also include a plasticizer such as glycerol or polyethylene glycol (m.w. 800 to 20,000) and penetrants such as azone. The composition of the carrier can be varied so long as it does not interfere with the pharmacological activity of the active ingredients.

The compositions may also include anti-microbial agents, other antiviral agents, anti-fungal agents, antioxidants, buffering agents, sunscreens and cosmetic agents such as toloring agents, fragrances, lubricants and moisturizers or drying agents. Anti-microbial agents useful for inclusion in the compositions include polymyxin B and tetracycline. Other antiviral agents included in the formulations may be nucleoside analogs such as acyclovir or cytokines. Anti-fungal agents that may be included are micatin or tolnaftate. Antioxidants such as vitamin E may be included. Sunscreens such as para-aminobenzoic acid may be included. Drying agents that may be included are well known, such as, for example, phenol and benzyl alcohol. Lubricants such as synthetic or natural beeswax may also be included. Thickening agents added to the compositions may include pullulin, xanthan, polyvinylpyrrolidone or carboxymethylcellulose.

Optimally the compositions effectively reduce the viral titre overall in the treated individual, particularly for systemic treatment, and in lesions, particularly for topical treatment of affected areas of the skin or mucous membrane. The disclosed methods of treatment also reduce symptoms of viral infection (e.g., pain associated with viral-caused lesions) and promote more rapid healing than seen without treatment.

The method of the present invention includes administration of a composition containing the active ingredient and a surfactant to a human or animal to treat or prevent viral infection. Administration is preferably to the skin or a mucous membrane using a cream, lotion, gel, ointment, suspension, aerosol spray or semi-solid formulation (e.g., a suppository), all formulated using methods well known in the art. Applications of the compositions containing the active ingredient and surfactant effective in preventing or treating a viral infection consist of one to ten applications of 10 mg to 10 g per application for one to fourteen days. Applications are generally once every twelve hours and up to once every four hours. Preferably two to four applications of the composition per day, of about 0.1 g to 5 g per application, for one to seven days are sufficient to prevent or treat a viral infection. For topical applications, the compositions are preferably applied to lesions daily as soon as symptoms (e.g., pain, swelling or inflammation) are detected.

The compositions and methods are useful for preventing or treating a variety of viral infections such as those caused by herpes viruses including HSV-1, HSV-2 and HSV-6, cytomegalovirus (CMV), Epstein-Barr virus (EBV) and varicella zoster virus (VZV), by influenza viruses, human lymphotrophic viruses (e.g., HTLV-1), human immunodeficiency viruses (e.g., HIV-1), papilloma virus and respiratory syncytial virus. Because of the cytostatic activity of some of the compositions, the compositions and methods are also useful for inhibiting malignant cell growth and/or metastasis. This cellular inhibition can be combined with well known treatments for cancer (e.g., irradiation and/or chemotherapy) to lead to total or partial remission of a tumor or other cancerous cell growth.

Unless defined otherwise, all scientific and technical terms used herein have the same meaning as commonly understood by those skilled in the relevant art. Unless mentioned otherwise, the techniques employed or contemplated herein are standard methodologies well known to one of ordinary skill in the art. The examples of embodiments are for illustration only.

### EXAMPLE 1

### Effects of Increasing the Ratio of Surfactant to Aliphatic Alcohol

The antiviral effect of increasing the ratio (w:w) of surfactant to aliphatic alcohol was demonstrated using increasing ratios of PLURONIC F-68® to *n*-docosanol (compare to Example 1 using a 1:1 (w:w) ratio of surfactant to alcohol). The 1:1 suspension has a molecular ratio of 26:1 for *n*-docosanol (m.w. 326.57) to surfactant (m.w. 8,400) molecules. Generally, increasing the amount of surfactant decreases the particle size in suspension and causes formation of smaller unilamellar, rather than multilamehar, vesicles (Sandra, A. and R. E. Pagano, *J. Biol. Chem.* 254:2244-2249, 1979). This results in more of the alcohol occurring at the particle surface where it is available for interaction with cells.

Suspensions of *n*-docosanol were made essential as described in Example 1 using a constant amount of the alcohol but increasing the amount of surfactant to achieve a 3:1, 5:1 and 10:1 (w:w) ratio of PLURONIC F-68® to *n*-docosanol in the final suspension. Increasing the surfactant to alcohol ratio increased the antiviral effectiveness of the suspension in Vero cell culture (FIG. 2). That is, the 3:1 surfactant to alcohol ratio suspension showed greater antiviral activity than the 1:1 ratio (at *n*-docosanol ≥ 8 mM); the 5:1 ratio suspension showed increased antiviral activity compared to the 1:1 ratio (at *n*-docosanol ≥ 4 mM); and the 10:1 ratio exhibited more antiviral activity compared to the 1:1 ratio (at *n*-docosanol ≥ 1 mM). The antiviral activity was dependent on the *n*-docosanol in the suspension because control cultures incubated with the same concentration of surfactant in suspension as for each of the ratio tested above showed essentially no antiviral activity (FIG. 2B).

The increased surfactant to alcohol ratio also correlated with an increase in the amount of cell-associated *n*-docosanol as determined using Vero cells incubated for 24 hours with surfactant*n*-[1-¹⁴C]docosanol suspensions. Cells incubated with suspensions containing a 4:1 ratio of surfactant to *n*-docasanol bound 7.8 x 10⁻⁶ µg/cell, whereas an equivalent culture incubated with a 1:1 ratio suspension bound 3.1 x 10⁻⁶ µg/cell. Optimal antiviral activity of *n*-docosanol was obtained with surfactant to alcohol ratios of about 4:1 to 5:1 (w:w).

The antiviral activity of the aliphatic compounds was not a property of a unique combination of the aliphatic compound and a particular nonionic surfactant in suspension. That is, other detergents produced effective antiviral suspensions of aliphatic alcohol. Suspensions of *n*-docosanol with a non-ionic octoxynol detergent (TRITON X-100®, Rohm & Haas) were prepared by: a) melting 2.5 g of *n*-docosanol with 1.5 g detergent at 90°C, b) mixing the melted solution with 500 ml saline at 90°C and 1.15 g polyvinylpyrrolidone (PVP), c) processing the hot mixture through a microfluidizer at 1300 psi for 5 cycles, and d) ultrafiltering the processed mixture through a hollow fiber cartridge to eliminate excess detergent and PVP. A control detergent suspension was prepared in a similar manner except that *n*-docosanol was omitted. Deoxycholate suspensions of *n*-docosanol (surfactant to alcohol ratio of 1:1 by weight) were prepared essentially as described above.

Both the octoxynol and deoxycholate suspensions of the *n*-docosanol inhibited HSV-2 plaque production in the Vero cell assay. Typical results are shown in FIG. 3. The octoxynol/*n*-docosanol suspension inhibited plaque formation relative to the octoxynol control at *n*-docosanol concentrations of greater than or equal to 2 mM with an EC₅₀ of about 4.5 mM. The nonionic surfactant used to make an aliphatic alcohol suspension does not account for the suspension's antiviral activity.

Increasing the ratio of surfactant to *n*-docosanol significantly increased the antiviral activity of the suspension. That is, the amount of *n*-docosanol in the suspension required for 50% inhibition of plaque production decreased (e.g., from 15 mM to 3 mM).

Although the present invention has been described in the context of particular examples and preferred embodiments, it will be understood that the invention is not limited to these embodiments and is defined by the claims that follow.

## Claims

1. A composition comprising a mixture of about 1:1 (w:w) to about 10:1 (w:w) of a nonionic surfactant and an active ingredient in a pharmaceutically acceptable diluent or carrier, wherein the nonionic surfactant is an octoxynol and wherein the active ingredient is stearyl alcohol, erucyl alcoholic, brassidyl alcohol, n-docosanol, arachidyl alcohol, n-docosane, n-docosanoic acid erucamide, stearic acid, or mixtures thereof.

2. A composition as claimed in claim 1, wherein the mixture comprises about 5:1 (w:w) to about 10:1 (w:w) of the nonionic surfactant and the active ingredient.

3. A composition as claimed in claim 1, wherein the mixture comprises about 4:1 (w:w) to about 10:1 (w:w) of the nonionic surfactant and stearic acid.

4. A composition as claimed in any one of the preceding claims, wherein the surfactant comprises a mixture of nonionic detergents.

5. A composition as claimed in claim 4, wherein the mixture of nonionic detergents comprises a defunctional block copolymer comprising a polyoxyalkylene derivative of propylene glycol having a molecular weight of about 25,000.

6. A composition as claimed in claim 4, wherein the mixture of nonionic detergents comprises a block polymer of ethylene oxide and propylene oxide having a molecular weight of about 8,400.

7. A composition as claimed in claim 4, wherein the mixture of nonionic detergents, comprises deoxycholate.

8. A composition as claimed in any one of the preceding claims, wherein the nonionic surfactant is octoxynol-9, octoxynol-10 or a combination thereof.

9. A composition as claimed in any one of the preceding claims, wherein the mixture comprises about 5:1 (w:w) of octoxynol and n-docosanoic acid.

10. A composition as claimed in any one of claims 1 to 8, wherein the mixture comprises about 5% to about 20% (w/w) stearic acid and further comprises a sugar based stearate.

11. A composition as claimed in any one of claims 1 to 8, wherein the mixture comprises about 10% to about 12% (w/w) of n-docosanol and further comprises a sugar based stearate.

12. A composition as claimed in any one of claims 1 to 8, wherein the mixture consists essentially of a suspension of a nonionic surfactant and stearic acid in a pharmaceutically acceptable diluent or carrier.

13. A composition as claimed in claim 12, wherein the nonionic surfactant is octoxynol-9 octoxynol-10 or a mixture thereof.

14. A composition as claimed in claim 1 or 2, wherein the active ingredient comprises n-docosanol.

15. A composition as claimed in claim 1, 2 or 14, wherein the mixture consists essentially of a suspension of a nonionic surfactant and n-docosanol in a pharmaceutically acceptable diluent or carrier.

16. A composition as claimed in claim 15, wherein the nonionic surfactant is octoxynol-9, octoxynol-10, or a combination thereof.

17. A composition as claimed in claim 14, wherein the mixture further comprises a sugar based stearate.

18. A composition comprising a mixture of about 1:1 (w:w) to about 10:1 (w:w) of an octoxynol and n-docosanol in a pharmaceutically acceptable diluent or carrier.

19. A composition as claimed in claim 18, wherein the octoxynol is octoxynol-9, octoxynol-10, or a combination thereof.

20. A composition as claimed in claim 18 or 19, wherein the mixture comprises about 5% to about 20% (w/w) of n-docosanol.

21. A composition as claimed in any one of claims 18 to 20, wherein the mixture comprises about 10% to about 12% (w/w) of n-docosanol.

22. A composition as claimed in any one of claims 18 to 21, wherein the mixture further comprises a sugar based stearate.

23. A composition as claimed in any one of claims 18 to 22, wherein the mixture comprises about 4:1 (w:w) to about 10:1 (w:w) of the octoxynol and n-docosanol.

24. A composition as claimed in any one of claims 18 to 23, wherein the mixture comprises about 5:1 (w:w) to about 10:1 (w:w) of the octoxynol and n-docosanol.

25. A composition as claimed in any one of claims 18 to 24, wherein the mixture comprises about 5:1 (w:w) of the octoxynol and n-docosanol.

26. A composition comprising a mixture as claimed in any one of claims 1 to 25, for use in inhibiting cell growth or proliferation.

27. A composition comprising a mixture as claimed in any one of claims 1 to 25, for use in prevention or treatment of a viral infection.

28. A composition as claimed in claim 27, wherein the viral infection is caused by a herpes simplex virus, cytomegalovirus, Epstein-Barr virus, varicella zoster virus, influenza virus, human lymphotrophic virus, human immunodeficiency virus, papilloma virus or respiratory syncytial virus.

29. A composition comprising a mixture as claimed in any one of claims 1 to 25, for use in relieving skin or membrane inflammation.

30. A composition comprising a mixture as claimed in any one of claims 1 to 25, for use in treating burns..

31. Use of the composition comprising a mixture as claimed in any one of claims 1 to 25, for preparation of a medicament suitable for topical, transembrane or parenteral application.

32. Use of the composition comprising a mixture as claimed in any one of claims 1 to 25, for preparation of a medicament for prevention or treatment of a viral infection.

33. Use of the composition comprising a mixture as claimed in any one of claims 1 to 25, for preparation of a medicament for treatment of burns.

## Patentansprüche

1. Zusammensetzung umfassend ein Gemisch von ca. 1:1 (w:w) bis ca. 10:1 (w:w) eines nicht ionischen Tensids und eines Wirkstoffs in einem pharmazeutisch verträglichen Verdünnungsmittel oder Träger, worin das nicht ionische Tensid ein Octoxynol darstellt und worin der Wirkstoff Stearylalkohol, Erucylalkohol, Brassidylalkohol, n-Docosanol, Arachidylalkohol, n-Docosan, n-Docosansäure Erucamid, Stearinsäure oder Gemische davon darstellt.

2. Zusammensetzung nach Anspruch 1, worin das Gemisch ca. 5:1 (w:w) bis ca. 10:1 (w:w) des nicht ionischen Tensids und des Wirkstoffs umfasst.

3. Zusammensetzung nach Anspruch 1, worin das Gemisch ca. 4:1 (w:w) bis ca. 10: 1 (w:w) des nicht ionischen Tensids und der Stearinsäure umfasst.

4. Zusammensetzung nach einem der vorangehenden Ansprüche, worin das Tensid ein Gemisch von nicht ionischen Detergenzien umfasst.

5. Zusammenfassung nach Anspruch 4, worin das Gemisch aus nicht ionischen Detergenzien ein defunktionales Blockcopolymer, umfassend ein Polyoxyalkylen-Derivat von Propylenglykol mit einem Molekulargewicht von ca. 25 000 umfasst.

6. Zusammensetzung nach Anspruch 4, worin das Gemisch aus nicht ionischen Detergenzien ein Blockcopolymer aus Ethylenoxid und Propylenoxid mit einem Molekulargewicht von ca. 8 400 umfasst.

7. Zusammensetzung nach Anspruch 4, worin das Gemisch aus nicht ionischen Detergenzien Desoxycholat umfasst.

8. Zusammensetzung nach einem der vorangehenden Ansprüche, worin das nicht ionische Tensid Octoxynol-9, Octoxynol-10 oder eine Kombination davon umfasst.

9. Zusammenfassung nach einem der vorangehenden Ansprüche, worin das Gemisch ca. 5:1 (w:w) Octoxynol und n-Docosansäure umfasst.

10. Zusammensetzung nach einem der Ansprüche 1 bis 8, worin das Gemisch ca. 5 % bis ca. 20 % (w/w) Stearinsäure umfasst und weiter ein auf Zucker basierendes Stearat umfasst.

11. Zusammensetzung nach einem der Ansprüche 1 bis 8, worin das Gemisch ca. 10 % bis ca. 12 % (w/w) n-Docosanol umfasst und weiter ein auf Zucker basierendes Stearat umfasst.

12. Zusammensetzung nach einem der Ansprüche 1 bis 8, worin das Gemisch im Wesentlichen aus einer Suspension eines nicht ionischen Tensids und einer Stearinsäure in einem pharmazeutisch verträglichen Verdünnungsmittel oder Träger besteht.

13. Zusammensetzung nach Anspruch 12, worin das nicht ionische Tensid Octoxynol-9, Octoxynol-10 oder ein Gemisch davon darstellt.

14. Zusammensetzung nach Anspruch 1 oder 2, worin der Wirkstoff n-Docosanol umfasst.

15. Zusammensetzung nach Ansprüchen 1, 2 oder 14, worin das Gemisch im Wesentlichen aus einer Suspension eines nicht ionischen Tensids und n-Docosanols in einem pharmazeutisch verträglichen Verdünnungsmittel oder Träger besteht.

16. Zusammensetzung nach Anspruch 15, worin das nicht ionische Tensid Octoxynol-9, Octoxynol-10 oder eine Kombination davon darstellt.

17. Zusammensetzung nach Anspruch 14, worin das Gemisch weiter ein auf Zucker basierendes Stearat umfasst.

18. Zusammensetzung umfassend ein Gemisch von ca. 1:1 (w:w) bis ca. 10: (w:w) eines Octoxynols und n-Docosanols in einem pharmazeutisch verträglichen Verdünnungsmittel oder Träger.

19. Zusammensetzung nach Anspruch 18, worin das Octoxynol Octoxynol-9, Octoxynol-10 oder eine Kombination davon darstellt.

20. Zusammensetzung nach Anspruch 18 oder 19, worin das Gemisch ca. 5 % bis ca. 20 % (w/w) n-Docosanol umfasst.

21. Zusammensetzung nach einem der Ansprüche 18 bis 20, worin das Gemisch ca. 10 % bis ca. 12 % (w/w) n-Docosanol umfasst.

22. Zusammensetzung nach einem der Ansprüche 18 bis 21, worin das Gemisch weiter ein auf Zucker basierendes Stearat umfasst.

23. Zusammensetzung nach einem der Ansprüche 18 bis 22, worin das Gemisch ca. 4:1 (w:w) bis ca. 10:1 (w:w) des Octoxynols und n-Docosanols umfasst.

24. Zusammensetzung nach einem der Ansprüche 18 bis 23, worin das Gemisch ca. 5:1 (w:w) bis ca. 10:1 (w:w) des Octoxynols und n-Docosanols umfasst.

25. Zusammensetzung nach einem der Ansprüche 18 bis 24, worin das Gemisch ca. 5:1 (w:w) des Octoxynols und n-Docosanols umfasst.

26. Zusammensetzung umfassend ein Gemisch nach einem der Ansprüche 1 bis 25 zur Verwendung bei der Inhibition des Wachstums oder der Proliferation von Zellen.

27. Zusammensetzung umfassend ein Gemisch nach einem der Ansprüche 1 bis 25 zur Verwendung bei der Prävention oder Behandlung einer Virusinfektion.

28. Zusammensetzung nach Anspruch 27, worin die Virusinfektion durch ein Herpessimplex-Virus, Cytomegalie-Virus, Epstein-Barr-Virus, Varicella-Zoster-Virus, Influenza-Virus, humanes lymphotrophes Virus, Human Immunodeficiency Virus, Papilloma-Virus oder Respiratory-Syncytial-Virus verursacht wird.

29. Zusammensetzung umfassend ein Gemisch nach einem der Ansprüche 1 bis 25 zur Verwendung bei der Linderung der Entzündung der Haut oder Membranen.

30. Zusammensetzung umfassend ein Gemisch nach einem der Ansprüche 1 bis 25 zur Verwendung bei der Behandlung von Verbrennungen.

31. Verwendung der Zusammensetzung umfassend ein Gemisch nach einem der Ansprüche 1 bis 25 zur Herstellung eines Arzneimittels, das zur topischen, transmembranösen oder parenteralen Applikation geeignet ist.

32. Verwendung der Zusammensetzung umfassend ein Gemisch nach einem der Ansprüche 1 bis 25 zur Herstellung eines Arzneimittels zur Prävention oder Behandlung einer Virusinfektion.

33. Verwendung der Zusammensetzung umfassend ein Gemisch nach einem der Ansprüche 1 bis 25 zur Herstellung eines Arzneimittels zur Behandlung von Verbrennungen.

## Revendications

1. Composition comprenant un mélange d'environ 1:1 (en poids) à environ 10:1 (en poids) d'un agent de surface non ionique et d'un ingrédient actif dans un diluant ou porteur pharmaceutiquement acceptable, dans laquelle l'agent de surface non ionique est un octoxynol et dans laquelle l'ingrédient actif est de l'alcool stéarylique, de l'alcool érucylique, de l'alcool brassidylique, du n-docosanol, de l'alcool arachidylique, du n-docosane, de l'érucamide d'acide n-docosanoïque, de l'acide stéarique ou des mélanges de ceux-ci.

2. Composition selon la revendication 1, dans laquelle le mélange comprend d'environ 5:1 (en poids) à environ 10:1 (en poids) de l'agent de surface non ionique et de l'ingrédient actif.

3. Composition selon la revendication 1, dans laquelle le mélange comprend d'environ 4:1 (en poids) à environ 10:1 (en poids) de l'agent de surface non ionique et d'acide stéarique.

4. Composition selon l'une quelconque des revendications précédentes, dans lequel l'agent de surface comprend un mélange de détergents non ioniques.

5. Composition selon la revendication 4, dans laquelle le mélange de détergents non ioniques comprend un copolymère bloc defonctionnel comprenant un dérivé de polyoxyalkylène de propylène glycol d'un poids moléculaire d'environ 25000.

6. Composition selon la revendication 4, dans laquelle le mélange de détergents non ioniques comprend un polymère bloc d'oxyde d'éthylène et d'oxyde de propylène d'un poids moléculaire d'environ 8400.

7. Composition selon la revendication 4, dans laquelle le mélange de détergents non ioniques comprend du déoxycholate.

8. Composition selon l'une quelconque des revendications précédentes, dans laquelle l'agent de surface non ionique est de l'octoxynol-9, de l'octoxynol-10 ou une combinaison de ceux-ci.

9. Composition selon l'une quelconque des revendications précédentes, dans laquelle le mélange comprend d'environ 5:1 (en poids) d'octoxynol et d'acide n-docosanoïque.

10. Composition selon l'une quelconque des revendications 1 à 8, dans laquelle le mélange comprend d'environ 5 % à environ 20 % (en poids) d'acide stéarique et comprend en outre un stéarate à base de sucre.

11. Composition selon l'une quelconque des revendications 1 à 8, dans laquelle le mélange comprend d'environ 10 % à environ 12 % (en poids) de n-docosanol et comprend en outre un stéarate à base de sucre.

12. Composition selon l'une quelconque des revendications 1 à 8, dans laquelle le mélange consiste essentiellement en une suspension d'un agent de surface non ionique et d'acide stéarique dans un diluant ou porteur pharmaceutiquement acceptable.

13. Composition selon la revendication 12, dans laquelle l'agent de surface non ionique est de l'octoxynol-9, de l'octoxynol-10 ou une combinaison de ceux-ci.

14. Composition selon la revendication 1 ou 2, dans laquelle l'ingrédient actif comprend du n-docosanol.

15. Composition selon la revendications 1, 2 ou 14, dans laquelle le mélange consiste essentiellement en une suspension d'un agent de surface non ionique et de n-docosanol dans un diluant ou porteur pharmaceutiquement acceptable.

16. Composition selon la revendication 15, dans laquelle l'agent de surface non ionique est de l'octoxynol-9, de l'octoxynol-10 ou une combinaison de ceux-ci.

17. Composition selon la revendication 14, dans laquelle le mélange comprend en outre un stéarate à base de sucre.

18. Composition comprenant un mélange d'environ 1:1 (en poids) à environ 10:1 (en poids) d'un octoxynol et de n-docosanol dans un diluant ou porteur pharmaceutiquement acceptable.

19. Composition selon la revendication 18, dans laquelle l'octoxynol est de l'octoxynol-9, de l'octoxynol-10 ou une combinaison de ceux-ci.

20. Composition selon la revendication 18 ou 19, dans laquelle le mélange comprend d'environ 5 % à environ 20 % (en poids) de n-docosanol.

21. Composition selon l'une quelconque des revendications 18 à 20, dans laquelle le mélange comprend d'environ 10 % à environ 12 % (en poids) de n-docosanol.

22. Composition selon l'une quelconque des revendications 18 à 21, dans laquelle le mélange comprend en outre un stéarate à base de sucre.

23. Composition selon l'une quelconque des revendications 18 à 22, dans laquelle le mélange comprend d'environ 4:1 (en poids) à environ 10:1 (en poids) d'octoxynol et de n-docosanol.

24. Composition selon l'une quelconque des revendications 18 à 23, dans laquelle le mélange comprend d'environ 5:1 (en poids) à environ 10:1 (en poids) d'octoxynol et de n-docosanol.

25. Composition selon l'une quelconque des revendications 18 à 24, dans laquelle le mélange comprend environ 5:1 (en poids) d'octoxynol et de n-docosanol.

26. Composition comprenant un mélange selon l'une quelconque des revendications 1 à 25, destinée à être utilisée pour inhiber une croissance ou prolifération cellulaire.

27. Composition comprenant un mélange selon l'une quelconque des revendications 1 à 25, destinée à être utilisée dans la prévention ou le traitement d'une infection virale.

28. Composition selon la revendication 27, dans laquelle l'infection virale est causée par un virus Herpès simplex, cytomégalovirus, virus d'Epstein-Barr, virus varicelle zona, virus Influenza, virus lymphotrophique humain, virus d'immunodéficience humaine, papilloma virus ou virus respiratoire syncytial.

29. Composition comprenant un mélange selon l'une quelconque des revendications 1 à 25, destinée à être utilisée pour apaiser une inflammation de la peau ou d'une membrane.

30. Composition comprenant un mélange selon l'une quelconque des revendications 1 à 25, destinée à être utilisée pour traiter les brûlures.

31. Utilisation de la composition comprenant un mélange selon l'une quelconque des revendications 1 à 25, pour la préparation d'un médicament pouvant être appliqué de façon topique, au travers d'une membrane ou de façon parentérale.

32. Utilisation de la composition comprenant un mélange selon l'une quelconque des revendications 1 à 25, pour la préparation d'un médicament pour la prévention ou le traitement d'une infection virale.

33. Utilisation de la composition comprenant un mélange selon l'une quelconque des revendications 1 à 25, pour la préparation d'un médicament servant au traitement de brûlures.
